(19) Europäisches Patentamt
European Patent Office
Office européen des brevets

(11) **EP 4 299 155 A1**

(12) **EUROPEAN PATENT APPLICATION**

(43) Date of publication:
**03.01.2024 Bulletin 2024/01**

(21) Application number: **22181207.6**

(22) Date of filing: **27.06.2022**

(51) International Patent Classification (IPC):
**B01D 3/00** *(2006.01)*   **B01D 3/42** *(2006.01)*
**B01D 3/10** *(2006.01)*   **C07C 67/03** *(2006.01)*
**C07C 67/54** *(2006.01)*   **B01D 3/14** *(2006.01)*

(52) Cooperative Patent Classification (CPC):
(C-Sets available)
**B01D 3/009; B01D 3/10; B01D 3/14; B01D 3/42;**
**C07C 67/03; C07C 67/54**                    (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
• **Evonik Operations GmbH**
  **45128 Essen (DE)**
• **Evonik Corporation**
  **Parsippany, NJ 07054-0677 (US)**

(72) Inventors:
• **TRESKOW, Marcel**
  **64293 Darmstadt (DE)**
• **LACKEY, Kevin**
  **Daphne, AL 36526 (US)**
• **COONROD, Mark**
  **Cantonment, FL 32533 (US)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(54) **PROCESS TO PRECISELY CALCULATE THE RAW MATERIAL DEMAND OF INTERMEDIATE BATCHES WITHIN A PRODUCTION CAMPAIGN**

(57) The present invention discloses a method for determining a demand amount of a reusable mixture, the reusable mixture comprises a first starting material or, if applicable, a second starting material, to provide the needed amount of the first starting material or, if applicable, the second starting material in a next batch reaction of a reaction mixture within a series of consecutive batch reactions of reaction mixtures in a reactor system, each batch reaction produces at a reaction temperature and a given pressure in the reaction chamber a batch of a product mixture comprising a first product and, if applicable, a second product, each reaction mixture comprises a first starting material and, if applicable, a second starting material, the reactor system comprises a reboiler, a reaction chamber comprising the reaction mixture, a column with a column head, a vapor transfer line, a condenser, a reflux tank, a reflux line, a distillate take off line with a mass flow meter, and a receiver vessel, wherein a reusable mixture is removed from the product mixture by distillate take off, the reusable mixture comprises at least a portion of the first starting material or, if applicable, of the second starting material, the removed reusable mixture is collected in the receiver vessel, the reusable mixture is reused to provide the needed amount of the first starting material or, if applicable, of the second starting material in the next batch reaction, characterized in that the demand amount of the reusable mixture comprising the first starting material or, if applicable, of the second starting material is determined by calculating, wherein parameter measured by the mass flow meter are used for the calculation.

Figure 1: Reactor system according to the invention, the reactor system comprises a reboiler (12), a reaction chamber (1), a column (2) with a column head (3), a vapor transfer line (4), a condenser (5), a reflux tank (6), a reflux line (7), a distillate take off line (8) with a mass flow meter (10), a receiver vessel (9), and a recycle line (11).

(52) Cooperative Patent Classification (CPC): (Cont.)

C-Sets
**C07C 67/03, C07C 69/54;**
**C07C 67/54, C07C 69/54**

## Description

### Field of the invention

The invention relates to the field of product production in a reactor by raw materials, where at the end of a reaction excess raw materials are separated from the product by distillation. These distilled excess raw materials are reused in further reaction cycles for producing the product. In particular, the invention relates to the determination of the raw material's amount.

### Background of the invention

During the production of products through reactions of raw materials, stoichiometric reactions are a very attractive goal from an environmental and economic perspective in order to save time and material. However, in reality at least one raw material is usually added in excess, especially if the reaction has a chemical equilibrium.

The excess raw material added to carry out the reaction is usually distilled off at the end of a reaction. The distilled excess raw material can be reused as raw material in the next batch to save material and costs. Ideally, the distilled excess raw material is pure and no further processing is required. For many or even for most products, the reality is different, and the distillate is a mixture of raw materials, by-products, and sometimes - especially in the case of volatile compounds - the products themselves. To account for these impurities, one usually assumes a purity of the excess raw material based on average production values.

However, this can become a serious process limitation if the assumed purity does not correspond to reality. Especially if the purity in the distillate is lower than assumed. In this case, there may be a shortage of raw materials in the next batch. This usually leads to a threatening, self-accelerating off-spec production, wherein products are not conforming to the specification, which is usually not discovered until the batches are finished and the product is analyzed.

To overcome this limitation, it is possible to measure and analyze the raw material "work in progress" (WIP, which is shorthand for the distilled raw material that is going to be reused) by taking samples of the distillate before each batch. However, this is a tremendous amount of work to pull, analyze and adjust the reaction composition while or before the new reaction process has already begun. Therefore, sampling is simply not suitable and does not work fast and reliably enough for daily operations.

The objective of the present invention is to provide a method for determining raw material quantities in a distillate that overcomes the aforementioned problems.

### Summary of the invention

The invention is a method for determining a demand amount of a reusable mixture, the reusable mixture comprises a first starting material or, if applicable, a second starting material, to provide the needed amount of the first starting material or, if applicable, the second starting material in a next batch reaction of a reaction mixture within a series of consecutive batch reactions of reaction mixtures in a reactor system, each batch reaction produces, at a reaction temperature and a given pressure in the reaction chamber, a batch of a product mixture comprising a first product and, if applicable, a second product, each reaction mixture comprises a first starting material and, if applicable, a second starting material, the reactor system comprises a reboiler, a reaction chamber comprising the reaction mixture, a column with a column head, a vapor transfer line, a condenser, a reflux tank, a reflux line, a distillate take off line with a mass flow meter, and a receiver vessel, wherein a reusable mixture is removed from the product mixture by distillate take off, the reusable mixture comprises at least a portion of the first starting material or, if applicable, of the second starting material, the removed reusable mixture is collected in the receiver vessel, the reusable mixture is reused to provide the needed amount of the first starting material or, if applicable, of the second starting material in the next batch reaction, characterized in that the demand amount of the reusable mixture comprising the first starting material or, if applicable, of the second starting material is determined by calculating, wherein parameter measured by the mass flow meter are used for the calculation.

To this end, the present invention utilizes a mass flow meter (MFM). The MFM determines the flowing volume and measures the density to calculate the flowing mass. Typically, temperature is also measured to correct for temperature-dependent volume change. To calculate the composition of a distillate, it is condensed at a certain temperature to obtain a composition which comprises at least 90 wt.-% of two components, the mentioned two components are considered to constitute a "binary mixture". The density of the mixture is determined by the MFM, considering volume contraction and temperature-dependent viscosity.

Based on the density ratio of a mixture of two compounds, the amount of raw material in the distillate can be determined and the amount of distillate required to use enough raw material in the next batch can be calculated.

**Description of the drawings**

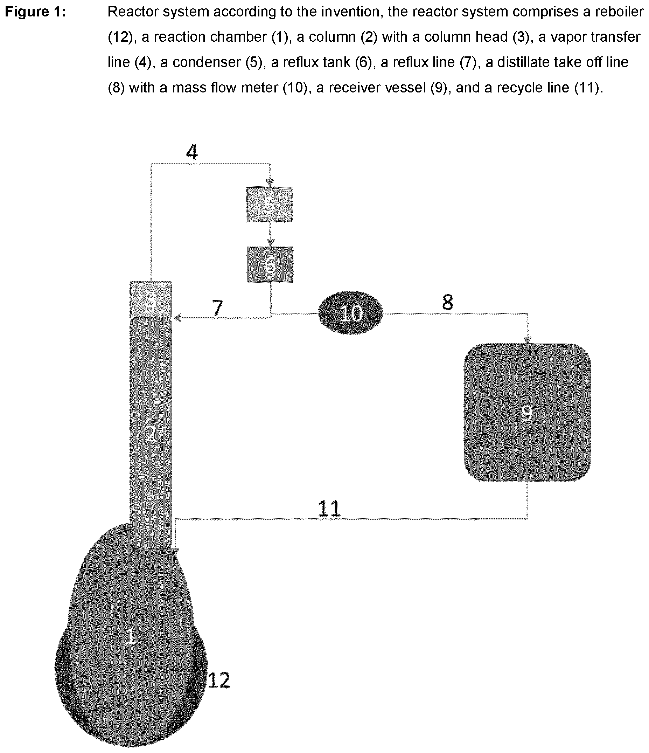

**Figure 1** illustrates a reactor system according to the invention, the reactor system comprises a reboiler (12), a reaction chamber (1), a column (2) with a column head (3), a vapor transfer line (4), a condenser (5), a reflux tank (6), a reflux line (7), a distillate take off line (8) with a mass flow meter (10), a receiver vessel (9), and a recycle line (11).

**Detailed description of the invention**

Unless otherwise particularly defined herein, the related terms used in the present invention have the following definitions.

As used herein, the term "reactor system" refers to a reactor in which a chemical reaction of a reaction mixture can take place, and wherein the contents of the reactor can be subjected to a distillation process before the course of a chemical reaction and/or during the course of a chemical reaction and/or after the course of a chemical reaction or independently of a chemical reaction. The advantage of such a reactor is that it is not necessary to provide several chambers, one for a reaction to take place and one for the substance or substances to be distilled, thus saving material, time for a transfer of the substances and costs. The reactor system comprises at least a reboiler, a reaction chamber, a column with a column head, a vapor transfer line, a condenser, a reflux tank, a reflux line, a distillate take off line with a mass flow meter, a receiver vessel, and, optionally, a recycle line.

As used herein, the term "reaction chamber level" refers to a volume level or filling level in the reaction chamber of the reactor system.

As used herein, the term "(trans)esterification" or "(trans)esterification reaction" refers to both an esterification reaction and a transesterification reaction. Or it refers to either an esterification reaction or a transesterification reaction depending on the respective context.

As used herein, the term "(meth)acrylate" refers to both (meth)acrylic acid and (meth)acrylic acid ester. Furthermore, it refers to both methacrylate and acrylate. Or it refers to methacrylate or acrylate depending on the respective context. It also refers to methacrylic acid and methacrylic acid ester. For example, the term "a (meth)acrylate compound" refers to (meth)acrylic acid and a (meth)acrylic acid ester, e.g. an alkyl (meth)acrylate.

As used herein, the term "starting material" refers to raw material, initial material, educts, feedstock, reactants or initial reactants which can be used to undergo a chemical reaction, whereby the chemical reaction may result at least in a product or products, which can include side-products or by-products.

As used herein, the term "reboiler" refers to an apparatus for heating a liquid and/or converting a liquid to its vapor or, in other words, gaseous state.

As used herein, the term "reaction chamber" refers to a container in which a chemical reaction is carried out. There are a wide variety of chambers being referred to as such. For example, the sizes of reaction chambers range from micro reaction chambers, which hold a few microliters, to reaction chambers for a few milliliters, to chambers with a volume of numerous cubic meters. The most important characteristic of each reaction chamber is its resistance to the reaction conditions.

As used herein, the term "reaction mixture" refers to substances that undergo a reaction. These substances can comprise or consist of the starting material, of the starting material and possible additives such as auxiliary substances, which can be, for example, catalysts or other reaction accelerators, of the starting material and products including possible side-products, of the starting material, additives and products including possible side-products, of the products including possible side-products and the additives, of the products alone and additives, of the products including possible side-products, or of the products alone.

As used herein, the term "column" refers to an apparatus for the thermal separation of mixtures. To avoid heat loss, the column can be an insulated, preferably cylindrical, tube, which can be made in particular of steel, high-alloy stainless steels, glass or plastic. The height of the column body can mainly be dictated by the required quality of separation; the diameter by the volume flow of the mixture to be separated. The column can be placed between the reaction chamber and the distillation head. The number of individual distillations required for the same separation performance can also be referred to as the "theoretical plate number". At the surface of the column, the equilibrium between the liquid and gaseous phases can constantly be re-established by condensation and evaporation. As a result, the proportion of the low-boiling component continues to increase towards the top, while the higher-boiling component flows back into the reaction chamber, the sump. The size of the surface area of the column can be greatly increased in various ways by the design of trays, as in the Vigreux column, or by filling with packing or structured packing.

As used herein, the term "feed line" refers to supply lines which feed substances or substance mixtures to e.g. the reaction chamber or to the column.

As used herein, the term "condenser" refers to a unit in which the vapor produced during distillation, which can be composed of the various volatile components of the solution to be separated, can liquefy by cooling.

As used herein, the term "reflux tank" refers to a container into which condensed distillate flows, which then either flows back to the column and/or into the reaction chamber or is removed from the system.

As used herein, the term "reflux line" refers to a line which can feed distillate from the reflux tank back to the column or to the reaction chamber.

As used herein, the term "distillate take off line" refers to a line which can remove at least a portion of distillate from the reflux tank, or in general from the reactor system, or the distillation system.

As used herein, the term "receiver vessel" refers to a container into which condensed distillate flows, which then either flows back to the column and/or into the reaction chamber or is removed from the system.

As used herein, the term "wt.-%" refers to weight percentage.

The problem underlying the present invention is solved by a method for determining a demand amount of a reusable mixture, the reusable mixture comprises a first starting material or, if applicable, a second starting material, to provide the needed amount of the first starting material or, if applicable, the second starting material in a next batch reaction of a reaction mixture within a series of consecutive batch reactions of reaction mixtures in a reactor system, each batch reaction produces at a reaction temperature and a given pressure in the reaction chamber a batch of a product mixture comprising a first product and, if applicable, a second product, each reaction mixture comprises a first starting material and, if applicable, a second starting material, the reactor system comprises a reboiler, a reaction chamber comprising the reaction mixture, a column with a column head, a vapor transfer line, a condenser, a reflux tank, a reflux line, a distillate take off line with a mass flow meter, and a receiver vessel, wherein a reusable mixture is removed from the product mixture by distillate take off, the reusable mixture comprises at least a portion of the first starting material or, if applicable, of the second starting material, the removed reusable mixture is collected in the receiver vessel, the reusable mixture is reused to provide the needed amount of the first starting material or, if applicable, of the second starting material in the next batch reaction, characterized in that the demand amount of the reusable mixture comprising the first starting material or, if applicable, of the second starting material is determined by calculating, wherein parameter measured by the mass flow meter are used for the calculation.

Removing and reusing a reusable mixture from the product mixture by distillate take off has the advantage that, especially at the end of a batch reaction, the batch reaction continues to run in the direction of product generation and does not come to a standstill. At the end of the batch reaction, the distillate is usually very rich in (e.g. high boiling) starting material(s). Furthermore, since the majority of the batch reaction is done, the following distillate take off is rich in (e.g. high boiling) valuable starting material too. It is therefore highly beneficial to save this take off, which comprises low amounts of side product and high amounts of starting material and return this excess portion of starting material that is removed at the end of each batch reaction to the next batch starting material.

Preferably, the product mixture comprises a first product and a second product.

Further preferably, the reaction mixture comprises two or more starting materials and two or more products. The two or more starting materials are a first educt, a second educt, and, optionally, one or more further compounds, and the two or more products are a first product, a second product, and, optionally, one or more further products. The one or more further compounds may be selected from further educts, solvents, catalysts, and/or additives.

The "reaction" in context of the method of the invention is preferably a (trans)esterification reaction for preparing a (meth)acrylate product.

If the reaction is a (trans)esterification reaction the reaction mixture may comprises an (meth)acrylate as first starting material and a first alcohol as second starting material which are converted by the (trans)esterification reaction into a second (meth)acrylate as a first product and a second alcohol (or water) as a second product.

The above-mentioned starting materials are preferably selected from the group consisting of alkyl (meth)acrylates, hydroxyalkyl (meth)acrylates, alkyl (meth)acrylate derivatives. Furthermore, the above-mentioned products are preferably selected from the group consisting of alkyl (meth)acrylates, and alkyl (meth)acrylate derivatives.

Examples for starting materials in context of the invention are methyl methacrylate (MMA), butyl methacrylate (BUMA), hydroxyethyl methacrylate (HEMA), and methacrylic acid (MAA).

Examples for products in context of the invention are selected from the group consisting of ethylene glycol dimethylacrylate (EGDMA), cetyl methacrylate (C16MA), magaryl methacrylate (C17MA), stearyl methacrylate (C18MA), myristyl methacrylate (C14MA), lauryl methacrylate (C12MA), benzyl methacrylate (BnMA), 1,4-butanediol dimethacrylate (1,4-BDDMA), 1,3-butanediol dimethacrylate (1,3-BDDMA), trimethylolpropane trimethacrylate (TMPTMA), polyethyleneglycol monomethylether methacrylate (MPEG methacrylate, having an average molecular weight from 250 g/mol up to 8000 g/mol), N-(2-methacryloyl oxyethyl) ethylene urea, and triethyleneglycol di(meth)acrylate, as well as mixtures of the above and natural or artificial distributions of the mentioned long chain alkyl groups.

In the method according to the invention, the alkyl (meth)acrylate starting materials may comprise methyl (meth)acrylate and the product may comprise methanol, or the alkyl (meth)acrylate starting material may comprise ethyl (meth)acrylate and the products may comprise ethanol, or the alkyl (meth)acrylate starting material may comprise n-butyl (meth)acrylate and the product may comprise butanol, or the alkyl (meth)acrylate starting material may comprise (meth)acrylic acid and the product may comprise water. Preferably, the alkyl (meth)acrylate starting material comprises methyl (meth)acrylate and the side product comprises methanol.

The reaction in the method of the invention preferably takes place in the presence of a catalyst.

In the method according to the invention, the catalyst can be selected from titanium(IV) alcoholates, for example titanium(IV) tetraisopropanolate, titanium(IV) tetrabutanolate, titanium(IV) tetrakis(2-ethylhexanolate), or mixtures thereof; from zirconium acetylacetonate; or from strong basic compounds, for example alkali oxides, earth alkali oxides, alkali hydroxides, earth alkali hydroxides, alkali alkoxides, earth alkali alkoxides, alkali amides, and earth alkali amides, preferably wherein the strong basic catalyst comprises one or more compounds selected from the group consisting of calcium oxide (CaO), calcium hydroxide (Ca(OH)$_2$), lithium hydroxide (LiOH), sodium methanolate (NaOMe), lithium methanolate (LiOMe), lithium *tert*-butoxide (LiOt-Bu), lithium *iso*-propoxide (LiOiPr), lithium amide (LiNH$_2$), or mixtures thereof.

In the method according to the invention, the removed reusable mixture is preferably flowing through the distillate take off line, and the parameter of the flowing reusable mixture are measured by the mass flow meter.

Preferably, the measured parameters of the flowing reusable mixture are volume, density, and, optionally, but preferably, temperature.

In the method according to the invention at least 90 wt.-%, preferably at least 95 wt.-%, more preferably at least 98 wt.-%, and most preferably at least 99 wt.-%, of the flowing reusable mixture constitute a "binary mixture" which consist of two components, namely the removed portion of the first starting material or, if applicable, of the second starting material and of one further component.

In preferred embodiments of the method of the invention, 95 wt.-% of the flowing reusable mixture consist of:

(i) methyl methacrylate (MMA) and methanol, or
(ii) methyl methacrylate (MMA) and ethylene glycol dimethacrylate (EGDMA), or
(iii) methyl methacrylate (MMA) and isopropyl methacrylate (IPMA).

In embodiments of the method of the invention the given pressure can be repeatedly adjusted over a predominant amount of time during one, or more than one, or all of the consecutive batch reactions in order to maintain a range of the reaction temperature. The given pressure is repeatedly adjusted in a way to maintain the reaction temperature typically in the range of from 70°C to not more than 160°C. Preferably, the given pressure is repeatedly adjusted in a way to maintain the reaction temperature in the range of from 80°C to not more than 150°C, more preferably in the range of from 90°C to not more than 145°C, for example in the range of from 115°C to 135°C.

In embodiments of the method of the invention the removal of the reusable mixture by distillate take off over a predominant amount of time during one, or more than one, or all of the consecutive batch reactions is a continuous removal.

According to the invention, it is preferred that the calculation for determining the demand amount of the reusable mixture uses the measured parameter to calculate a mass fraction of each of the two components of the binary mixture relative to the total mass of the binary mixture.

In the method according to the invention, the calculation for determining the demand amount of the reusable mixture can use the measured parameter to calculate a mass fraction of each of two components of a first binary mixture relative to the total mass of the first binary mixture and to calculate a mass fraction of each of two components of a second binary mixture relative to the total mass of the second binary mixture, wherein the second binary mixture starts to flow at a certain pressure during the distillation.

Alternatively, in the method according to the invention, the calculation for determining the demand amount of the reusable mixture can use the measured parameter to calculate a mass fraction of each of two components of a first binary mixture relative to the total mass of the first binary mixture and to calculate a mass fraction of each of two components of a second binary mixture relative to the total mass of the second binary mixture, wherein the second binary mixture starts to flow at a certain density threshold of the flowing mixture.

The following compilation of calculations as well as the following examples aim to explain and clarify to the skilled person the principles of the present invention and to show how the invention can be applied in practice.

**Compilation of calculations**

The fraction of a compound in a mixture is equal to its mass divided by the total mass of the mixture as every chemist get taught during education.

$$(1) \qquad x_1 = \frac{m_1}{m_g}$$

Looking at a binary mixture it is obvious that

$$(2) \qquad m_g = m_1 + m_2$$

since every textbook teaches $\rho_i = \dfrac{m_i}{V_i}$ and therefore $V_i\,\rho_i = m_i$ this can be transformed to the term

$$(3) \qquad V_1\,\rho_1 + V_2\,\rho_2 = V_g\,\rho_g$$

At this point, one needs to consider that the binary mixture is an ideal mixture and assume that the error of volume contraction is negligible for the purpose of this invention, and assume $V_1 + V_2 = V_g$ which gives for the term above

$$(4) \qquad V_1\,\rho_1 + V_2\,\rho_2 = \rho_g\,(V_1 + V_2)$$

with the operation |: $V_1$

$$(5) \qquad \rho_1 + \rho_2\,\frac{V_2}{V_1} = \rho_g\left(1 + \frac{V_2}{V_1}\right)$$

directly gives

$$(6) \qquad \rho_1 + \rho_2\,\frac{V_2}{V_1} = \rho_g + \rho_g\,\frac{V_2}{V_1}$$

and with the operations $|{-}\rho_1$ and $-\rho_g\dfrac{V_2}{V_1}$ this transform to

$$(7) \qquad \rho_2\,\frac{V_2}{V_1} - \rho_g\,\frac{V_2}{V_1} = \rho_g - \rho_1$$

isolating the densities gives intermediately

$$(8) \qquad \frac{V_2}{V_1}\,(\rho_2 - \rho_g) = \rho_g - \rho_1$$

and this finally with operation |: $(\rho_2 - \rho_g)$

$$(9) \qquad \frac{V_2}{V_1} = \frac{\rho_g - \rho_1}{\rho_2 - \rho_g}$$

which explains the relation of the volume by the pure and mixed densities.

From here, a second point of view is required to set this into relation for the mass fraction, so we are looking back to equation (1) and (2) which easily merge to (10) $x_1 = \dfrac{m_1}{m_1 + m_2}$ since still every textbook teaches $\rho_i = \dfrac{m_i}{V_i}$ and therefore $V_i\,\rho_i = m_i$ this transformed to the term

$$(11) \quad x_1 = \frac{V_1\,\rho_1}{V_1\,\rho_1 + V_2\,\rho_2}$$

by extension of the term $\left|* \dfrac{\frac{1}{V_1}}{\frac{1}{V_1}}\right.$ this results in

$$(12) \quad x_1 = \frac{\rho_1}{\rho_1 + \rho_2 \frac{V_2}{V_1}}$$

with relation $\dfrac{V_2}{V_1} = \dfrac{\rho_g - \rho_1}{\rho_2 - \rho_g}$ developed in equation (9) the target function is already described, which explains the mass fraction only relation of densities

$$(13) \quad x_1 = \frac{\rho_1}{\rho_1 + \rho_2 \frac{\rho_g - \rho_1}{\rho_2 - \rho_g}}$$

Here it is object to the observer whether it is necessary to simplify the equation, to obtain something handier to deal with.

With focus on the denominator

i. $\rho_1 + \rho_2 \dfrac{\rho_g - \rho_1}{\rho_2 - \rho_g}$ extended with $\rho_2 - \rho_g$ gives

ii. $\dfrac{\rho_1(\rho_2 - \rho_g) + \rho_2(\rho_g - \rho_1)}{\rho_2 - \rho_g}$ resolving the brackets leads to

iii. $\dfrac{\rho_1\rho_2 - \rho_1\rho_g + \rho_2\rho_g - \rho_1\rho_2}{\rho_2 - \rho_g}$ which simplifies to

iv. $\dfrac{-\rho_1\rho_g + \rho_2\rho_g}{\rho_2 - \rho_g}$ and furthermore to

v. $\dfrac{(-\rho_1 + \rho_2)\rho_g}{\rho_2 - \rho_g}$ which getting back to (13) give

$$(14) \quad x_1 = \frac{\rho_1}{\frac{(-\rho_1 + \rho_2)\rho_g}{\rho_2 - \rho_g}}$$

which than simplifies to

$$(15) \quad x_1 = \frac{\rho_1(\rho_2 - \rho_g)}{(\rho_2 - \rho_1)\rho_g}$$

and finally, to the handy equation

$$(16) \quad x_1 = \frac{-\rho_1 + \frac{\rho_1\rho_2}{\rho_g}}{(\rho_2 - \rho_1)}$$

With the assumption made before that the mixture is only binary, the fraction of the second compound is directly available as

$$(17) \quad x_2 = 1 - x_1$$

With that the distillate is fully described, assuming that there are no major impurities beside the defined binary mixture. Preferred <5%, more preferred <2%, much more preferred <1%, and most preferred <0.5% of impurities beside the define mixture. A further limitation build into the logic is that the values of X1 and X2 are limited to the range between 0 to 100% (in fact 0 to 1) and any result out of this region is return as the max or min value respectively. Since the mass flow meter measures mass flow, density and temperature, the mass flowing per time with density and temperature is known at any moment.

A single point of measurement gives the total mass flow ($\dot{m}_g$) from the flowmeter in mass / time, like $\dot{m}_g \frac{lbs}{min}$ or $\dot{m}_g \frac{kg}{h}$ etc. However, this flow divides for a mixture in the flow for each component ($\dot{m}_i$).

$$(18) \quad \dot{m}_g = \sum \dot{m}_i$$

Which simplifies again for a binary mixture to

$$(19) \quad \dot{m}_g = \dot{m}_1 + \dot{m}_2$$

and therefore to

$$(20) \quad \dot{m}_g = \dot{m}_g * x_1 + \dot{m}_g * x_2$$

which results in

$$(21) \quad \dot{m}_1 = \dot{m}_g * x_1 \ and \ \dot{m}_2 = \dot{m}_g * x_2$$

this directly gives together with equation (13) the flow per component at the moment of measurement.

$$(22) \quad \dot{m}_1 = \dot{m}_g * \frac{-\rho_1 + \frac{\rho_1 \rho_2}{\rho g}}{(\rho_2 - \rho_1)}$$

Building the integral of this function over time describes the amount of the compound totally flown during the process of distillation.

$$(23) \quad \int_{start}^{end} f(t)dt \ \dot{m}_1 = \sum m_1 \ ,$$

which is luckily simple mathematical operation for flow meter and called totalizer.

Repeating this mass count with all components of all binary systems and under continuation of totalizer for identical compounds, the distillate can be reliably count and calculated which replaces a purity measurement for the distillate.

**Examples**

Example 1:

Calculation of the mass fraction of the system methanol (MeOH) and MMA at 20°C, where the measured density of the mixture is 0.840 g/cm$^3$.

Density of MMA at 20°C = 0.940 g/cm$^3$
Density of MeOH at 20°C = 0.791 g/cm$^3$

As was previously explained:

$$x_1 = \frac{-\rho_1 + \frac{\rho_1 \rho_2}{\rho g}}{(\rho_2 - \rho_1)}$$

$$x_1 = \frac{-0.791 + \left(\frac{0.94 \ast 0.791}{0.84}\right)}{(0.94 - 0.791)} = \frac{-0.791 + \frac{0.74354}{0.84}}{0.149}$$

$$= \frac{-0.791 + 0.885}{0.149} = \frac{0.094167}{0.149} = 0.631991$$

$x_1$ = 0.63199 or the methanol fraction of this condensate stream is 63.20%
with equation (17) $x_2 = 1 - x_1$ the remaining component $x_2$ = 0.36801 or 36.80% MMA.

Example 2:

During the batch distillation of crude ethylene glycol dimethacrylate in which MMA is removed, several samples were taken and the calculated DCS result was compared to the chemical analysis. Furthermore, the finally collected fractions were sampled and their result was compared to their analysis and, finally, the overall calculated purity was compared to the total amount of distillate. The calculation method between the three compounds during this distillation was changed once the density exceeded the density of pure MMA.

| Sample | Sample origin | Analysis | | | DCS Calculation | | |
|---|---|---|---|---|---|---|---|
| | | **MEOH [%]** | **MMA [%]** | **EGDMA [%]** | **MEOH [%]** | **MMA [%]** | **Product [%]** |
| 1 | | 2.97 | 96.99 | 0.01 | 3.31 | 96.69 | 0.00 |
| 2 | | 1.19 | 98.75 | 0.00 | 1.50 | 98.50 | 0.00 |
| 3 | | 0.41 | 99.30 | 0.17 | 0.43 | 99.57 | 0.00 |
| 4 | | 0.20 | 94.27 | 5.14 | 0.00 | 94.53 | 5.47 |
| 5 | | 0.15 | 95.62 | 3.77 | 0.00 | 96.13 | 3.87 |
| 6 | 1 st fraction (vessel 1) | 0.67 | 98.94 | 0.31 | 0.74 | 99.00 | 0.26 |
| 7 | 2nd fraction (vessel 2) | 0.26 | 94.36 | 4.90 | 0.00 | 95.02 | 4.98 |
| 8 | **total** | 0.61 | 96.30 | 2.94 | 0.49 | 96.70 | 2.81 |

The calculated purity is in superior agreement with the analytical result, and its preciseness is within 0.5% of the analytical results.

Example 3:

During the batch distillation of crude Ethylene glycol dimethacrylate in which MMA is removed, several samples were taken and the calculated DCS result was compared to the chemical analysis. Additionally, the vessel containing the distillate was sampled and its result was compared to the analysis. Due to the reduced amount of MMA collected, only one vessel was used for total volume. The calculation method between the three compounds during this distillation was changed, once the initial pressure at which the distillation started was reduced by 50%. With a starting pressure of 9 PSIA, the calculation changed at 4.5 PSIA.

| Sample | Sample origin | Analysis | | | DCS Calculation | | | Pressur e |
|---|---|---|---|---|---|---|---|---|
| | | **MEOH [%]** | **MMA [%]** | **Product [%]** | **MEOH [%]** | **MMA [%]** | **Product [%]** | **[psi]** |
| 1 | | 3.21 | 96.99 | 0.00 | 3.52 | 96.48 | 0.00 | 7.1 |
| 2 | | 1.25 | 98.42 | 0.00 | 1.50 | 98.50 | 0.00 | 5.8 |
| 3 | | 0.36 | 99.32 | 0.09 | 0.00 | 100.00 | 0.00 | 2.9 |
| 4 | | 0.23 | 94.35 | 4.87 | 0.00 | 95.29 | 4.71 | 1.5 |
| 5 | | 0.11 | 94.62 | 5.12 | 0.00 | 95.02 | 4.98 | 0.7 |
| 6 | 1 st fraction (vessel 1) | 0.67 | 98.85 | 0.11 | 0.92 | 99.08 | 0.00 | |
| 7 | 2nd fraction (vessel 2) | 0.26 | 94.36 | 4.58 | 0.00 | 95.19 | 4.81 | |
| 8 | total | 0.61 | 96.10 | 2.94 | 0.45 | 96.54 | 3.01 | |

The calculated purity is in excellent agreement with the analytical result and its preciseness is within 0.5% of the analytical results.

Example 4:

During the batch distillation of crude benzyl methacrylate, which was made with the catalyst tetraisopropyltitanate, MMA is removed and next to methanol and product a further compound of isopropylmethacrylate (IPMA) is observed. Several samples were taken and the calculated DCS result was compared to the chemical analysis. Additionally, the vessel containing the distillate were sampled and their result was compared to their analyses and, finally, the overall calculated purity was compared to analytical result of the total amount of distillate.

The calculation method between the three compounds during this distillation was changed from (MeOH / MMA) to (IPMA / MMA) once the density of the condensate exceeds the density of MMA and then after the expiration of 15 minutes time to the binary mixture (product / MMA).

| Sample | Analysis | | | | DCS Calculation | | | |
|---|---|---|---|---|---|---|---|---|
| | **MEOH** [%] | **IPMA [%]** | **MMA [%]** | **BnMA [%]** | **MEOH [%]** | **IPMA [%]** | **MMA [%]** | **Product [%]** |
| 1 | 0.95 | 0.02 | 98.80 | 0.07 | 0.78 | 0.00 | 99.22 | 0.00 |
| 2 | 0.45 | 0.67 | 98.47 | 0.24 | 0.00 | 0.79 | 99.21 | 0.00 |
| 3 | 0.10 | 0.84 | 97.52 | 1.29 | 0.00 | 2.14 | 97.86 | 0.00 |
| 4 | 0.16 | 0.02 | 94.51 | 4.11 | 0.00 | 0.00 | 95.99 | 4.01 |
| 5 | 0.10 | 0.02 | 90.38 | 7.93 | 0.00 | 0.00 | 92.14 | 7.86 |
| 6 | 0.07 | 0.02 | 77.38 | 20.23 | 0.00 | 0.00 | 79.94 | 20.06 |
| 7 (total) | 1.37 | 0.41 | 93.54 | 4.40 | 1.13 | 0.35 | 93.90 | 4.62 |

Even a quaternary system can be calculated very well with the given assumptions and simplifications. However, the intermediate error between within single samples is increasing with complexity. Nevertheless, the overall purity is again in superior agreement with the analytics.

**Claims**

1. Method for determining a demand amount of a reusable mixture, the reusable mixture comprises a first starting material or, if applicable, a second starting material, to provide the needed amount of the first starting material or, if applicable, the second starting material in a next batch reaction of a reaction mixture within a series of consecutive batch reactions of reaction mixtures in a reactor system, each batch reaction produces at a reaction temperature and a given pressure in the reaction chamber a batch of a product mixture comprising a first product and, if applicable, a second product, each reaction mixture comprises a first starting material and, if applicable, a second starting material, the reactor system comprises a reboiler, a reaction chamber comprising the reaction mixture, a column with a column head, a vapor transfer line, a condenser, a reflux tank, a reflux line, a distillate take off line with a mass flow meter, and a receiver vessel, wherein a reusable mixture is removed from the product mixture by distillate take off, the reusable mixture comprises at least a portion of the first starting material or, if applicable, of the second starting material, the removed reusable mixture is collected in the receiver vessel, the reusable mixture is reused to provide the needed amount of the first starting material or, if applicable, of the second starting material in the next batch reaction, **characterized in that** the demand amount of the reusable mixture comprising the first starting material or, if applicable, of the second starting material is determined by calculating, wherein parameter measured by the mass flow meter are used for the calculation.

2. Method according to claim 1, wherein the product mixture comprises a first product and a second product.

3. Method according to one or more of the preceding claims, wherein the removed reusable mixture is flowing through the distillate take off line, and wherein the parameter of the flowing reusable mixture are measured by the mass flow meter.

4. Method according to one or more of the preceding claims, wherein the measured parameter of the flowing reusable mixture are volume, density, and temperature.

5. Method according to claim 2 or 3, wherein at least 90 wt.-%, preferably 95 wt.-%, more preferably 98 wt.-%, and most preferably 99 wt.-%, of the flowing reusable mixture constitute a binary mixture which consist of two components, namely the removed portion of the first starting material or, if applicable, of the second starting material and of one further component.

6. Method according to claim 5, wherein 95 wt.-% of the flowing reusable mixture consist of:

   (i) methyl methacrylate and methanol, or
   (ii) methyl methacrylate and ethylene glycol dimethacrylate, or
   (iii) methyl methacrylate and isopropyl methacrylate.

7. Method according to one or more of the preceding claims, wherein over a predominant amount of time during one, or more than one, or all of the consecutive batch reactions the given pressure is repeatedly adjusted in order to maintain a range of the reaction temperature, preferably wherein the given pressure is repeatedly adjusted in a way to maintain the reaction temperature in the range of from 70°C to not more than 160°C, preferably in the range of from 80°C to not more than 150°C, more preferably in the range of from 90°C to not more than 145°C.

8. Method according to one or more of the preceding claims, wherein over a predominant amount of time during one, or more than one, or all of the consecutive batch reactions the removal of the reusable mixture by distillate take off is a continuous removal.

9. Method according to one or more of the claims 5 to 8, wherein the calculation for determining the demand amount of the reusable mixture uses the measured parameter to calculate a mass fraction of each of the two components of the binary mixture relative to the total mass of the binary mixture.

10. Method according to one or more of the claims 5 to 9, wherein the calculation for determining the demand amount of the reusable mixture uses the measured parameter to calculate a mass fraction of each of two components of a first binary mixture relative to the total mass of the first binary mixture and to calculate a mass fraction of each of two components of a second binary mixture relative to the total mass of the second binary mixture, wherein the second binary mixture starts to flow at a certain pressure during the distillation.

11. Method according to one or more of the claims 5 to 9, wherein the calculation for determining the demand amount of the reusable mixture uses the measured parameter to calculate a mass fraction of each of two components of a first binary mixture relative to the total mass of the first binary mixture and to calculate a mass fraction of each of two components of a second binary mixture relative to the total mass of the second binary mixture, wherein the second binary mixture starts to flow at a certain density threshold of the flowing mixture.

**Figure 1:** Reactor system according to the invention, the reactor system comprises a reboiler (12), a reaction chamber (1), a column (2) with a column head (3), a vapor transfer line (4), a condenser (5), a reflux tank (6), a reflux line (7), a distillate take off line (8) with a mass flow meter (10), a receiver vessel (9), and a recycle line (11).

Europäisches
Patentamt

European
Patent Office

Office européen
des brevets

# EUROPEAN SEARCH REPORT

Application Number

EP 22 18 1207

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2012/071158 A1 (ROHM & HAAS [US]; CHALFANT DAVID [US] ET AL.) 31 May 2012 (2012-05-31) * figure 1 * * paragraphs [0020], [0022], [0041] * | 1-11 | INV. B01D3/00 B01D3/42 B01D3/10 C07C67/03 C07C67/54 B01D3/14 |
| Y | US 2014/027266 A1 (HOEHN RICHARD K [US] ET AL) 30 January 2014 (2014-01-30) * paragraph [0020] * | 1-11 | |
| Y | US 3 259 734 A (MARR JR GEORGE R) 5 July 1966 (1966-07-05) * column 3, lines 53-55 * | 1-11 | |
| A | EP 0 916 643 A1 (ROHM & HAAS [US]) 19 May 1999 (1999-05-19) * abstract * * figure 1 * * paragraph [0014] * | 1-11 | |
| A | EP 3 015 449 A1 (OSAKA ORGANIC CHEMICAL IND LTD [JP]) 4 May 2016 (2016-05-04) * figures 1, 2 * * abstract * | 1-11 | **TECHNICAL FIELDS SEARCHED (IPC)** B01D C07C |
| A | US 2013/172598 A1 (KNEBEL JOACHIM [DE] ET AL) 4 July 2013 (2013-07-04) * paragraphs [0035] - [0038] * | 1-11 | |
| A | WO 2009/080380 A2 (EVONIK ROEHM GMBH [DE]; KNEBEL JOACHIM [DE] ET AL.) 2 July 2009 (2009-07-02) * page 10, lines 1-26 * | 1-11 | |
| A | US 2010/204509 A1 (PROTZMANN GUIDO [DE] ET AL) 12 August 2010 (2010-08-12) * paragraph [0045] * | 1-11 | |

The present search report has been drawn up for all claims

| Place of search | Date of completion of the search | Examiner |
|---|---|---|
| The Hague | 22 December 2022 | Van Ganswijk, J |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   .................................................................
& : member of the same patent family, corresponding
   document

EPO FORM 1503 03.82 (P04C01)

# EP 4 299 155 A1

## ANNEX TO THE EUROPEAN SEARCH REPORT
## ON EUROPEAN PATENT APPLICATION NO.

EP 22 18 1207

This annex lists the patent family members relating to the patent documents cited in the above-mentioned European search report.
The members are as contained in the European Patent Office EDP file on
The European Patent Office is in no way liable for these particulars which are merely given for the purpose of information.

22-12-2022

| Patent document cited in search report | | | Publication date | Patent family member(s) | | | Publication date |
|---|---|---|---|---|---|---|---|
| WO 2012071158 | A1 | | 31-05-2012 | BR | 112013012674 | A2 | 13-09-2016 |
| | | | | CN | 103221379 | A | 24-07-2013 |
| | | | | EP | 2643288 | A1 | 02-10-2013 |
| | | | | JP | 6030564 | B2 | 24-11-2016 |
| | | | | JP | 2014506235 | A | 13-03-2014 |
| | | | | KR | 20140003445 | A | 09-01-2014 |
| | | | | SG | 190284 | A1 | 28-06-2013 |
| | | | | TW | 201229029 | A | 16-07-2012 |
| | | | | US | 2013245309 | A1 | 19-09-2013 |
| | | | | WO | 2012071158 | A1 | 31-05-2012 |
| US 2014027266 | A1 | | 30-01-2014 | NONE | | | |
| US 3259734 | A | | 05-07-1966 | GB | 1021470 | A | 02-03-1966 |
| | | | | US | 3259734 | A | 05-07-1966 |
| EP 0916643 | A1 | | 19-05-1999 | BR | 9804644 | A | 01-02-2000 |
| | | | | CA | 2252748 | A1 | 17-05-1999 |
| | | | | CN | 1218029 | A | 02-06-1999 |
| | | | | DE | 69803027 | T2 | 20-06-2002 |
| | | | | EP | 0916643 | A1 | 19-05-1999 |
| | | | | ES | 2169484 | T3 | 01-07-2002 |
| | | | | JP | 4111254 | B2 | 02-07-2008 |
| | | | | JP | H11236352 | A | 31-08-1999 |
| | | | | KR | 19990045236 | A | 25-06-1999 |
| | | | | TW | 422833 | B | 21-02-2001 |
| EP 3015449 | A1 | | 04-05-2016 | CN | 105339341 | A | 17-02-2016 |
| | | | | EP | 3015449 | A1 | 04-05-2016 |
| | | | | JP | 6363599 | B2 | 25-07-2018 |
| | | | | JP | WO2014208686 | A1 | 23-02-2017 |
| | | | | KR | 20160026829 | A | 09-03-2016 |
| | | | | US | 2016136538 | A1 | 19-05-2016 |
| | | | | WO | 2014208686 | A1 | 31-12-2014 |
| US 2013172598 | A1 | | 04-07-2013 | AU | 2008271502 | A1 | 08-01-2009 |
| | | | | BR | PI0814406 | A2 | 27-01-2015 |
| | | | | CA | 2692578 | A1 | 08-01-2009 |
| | | | | CN | 101337888 | A | 07-01-2009 |
| | | | | DE | 102007031473 | A1 | 08-01-2009 |
| | | | | EP | 2162419 | A1 | 17-03-2010 |
| | | | | ES | 2505342 | T3 | 09-10-2014 |
| | | | | JP | 5496090 | B2 | 21-05-2014 |
| | | | | JP | 2010532329 | A | 07-10-2010 |
| | | | | KR | 20100036302 | A | 07-04-2010 |
| | | | | RU | 2010103713 | A | 10-08-2011 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 22 18 1207

This annex lists the patent family members relating to the patent documents cited in  the above-mentioned European search report.
The members are as contained in the European Patent Office EDP file on
The European Patent Office is in no way liable for these particulars which are merely given for the purpose of information.

22-12-2022

| Patent document cited in search report | Publication date | Patent family member(s) | | Publication date |
|---|---|---|---|---|
| | | TW | 200909410 A | 01-03-2009 |
| | | US | 2013172598 A1 | 04-07-2013 |
| | | WO | 2009003745 A1 | 08-01-2009 |
| WO 2009080380 A2 | 02-07-2009 | CN | 101462958 A | 24-06-2009 |
| | | EP | 2220023 A2 | 25-08-2010 |
| | | JP | 2011506517 A | 03-03-2011 |
| | | JP | 2014237723 A | 18-12-2014 |
| | | JP | 2017088608 A | 25-05-2017 |
| | | JP | 2020073555 A | 14-05-2020 |
| | | JP | 2022081593 A | 31-05-2022 |
| | | TW | 200946496 A | 16-11-2009 |
| | | US | 2010280205 A1 | 04-11-2010 |
| | | US | 2016046556 A1 | 18-02-2016 |
| | | WO | 2009080380 A2 | 02-07-2009 |
| US 2010204509 A1 | 12-08-2010 | AU | 2008271501 A1 | 08-01-2009 |
| | | BR | PI0814012 A2 | 03-02-2015 |
| | | CA | 2692615 A1 | 08-01-2009 |
| | | CN | 101337889 A | 07-01-2009 |
| | | DE | 102007031470 A1 | 08-01-2009 |
| | | EP | 2162423 A1 | 17-03-2010 |
| | | JP | 2010532328 A | 07-10-2010 |
| | | KR | 20100036303 A | 07-04-2010 |
| | | RU | 2010103714 A | 10-08-2011 |
| | | TW | 200909409 A | 01-03-2009 |
| | | US | 2010204509 A1 | 12-08-2010 |
| | | WO | 2009003744 A1 | 08-01-2009 |

EPO FORM P0459

For more details about this annex : see Official Journal of the European Patent Office, No. 12/82

page 2 of 2